(19) Europäisches Patentamt
European Patent Office
Office européen des brevets

(11) Veröffentlichungsnummer: **0 044 008**
**B1**

(12) # EUROPÄISCHE PATENTSCHRIFT

(45) Veröffentlichungstag der Patentschrift: **05.12.84**

(51) Int. Cl.³: **C 07 D 263/58**, C 07 D 277/68, A 01 N 43/76, A 01 N 43/78

(21) Anmeldenummer: **81105203.4**

(22) Anmeldetag: **04.07.81**

(54) **Verwendung von 2-Propargyloxy-benzazolen als Synergisten.**

(30) Priorität: **16.07.80 DE 3026957**
**31.10.80 DE 3041152**

(43) Veröffentlichungstag der Anmeldung: **20.01.82 Patentblatt 82/3**

(45) Bekanntmachung des Hinweises auf die Patenterteilung: **05.12.84 Patentblatt 84/49**

(84) Benannte Vertragsstaaten: **AT BE CH DE FR GB IT LI NL SE**

(56) Entgegenhaltungen:
**US - A - 2 769 010**

**CHEMICAL ABSTRACTS, Band 91, Heft 1,2. Juli 1979, Seite 149, Zusammenfassung 1354j, COLUMBUS, OHIO (US)**
**CHEMICAL ABSTRACTS, Band 89, Heft 3, 17. Juli 1978, Seite 642, Zusammenfassung 24202f, COLUMBUS, OHIO (US) Y. WATANABE et al.: "An efficient phosphorylaton method by the activation of alcohol"**
**CHEMICAL ABSTRACTS, Band 93, Heft 15, 3. Oktober 1980, Seite 719, Zusammenfassung 150238t, COLUMBUS, OHIO (US)**

(73) Patentinhaber: **BAYER AG, Konzernverwaltung RP Patentabteilung, D-5090 Leverkusen 1 Bayerwerk (DE)**

(72) Erfinder: **Mues, Volker, Dr., Gellertweg 13, D-5600 Wuppertal 1 (DE)**
Erfinder: **Behrenz, Wolfgang, Dr., Untergründemich 14, D-5063 Overath (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft die Verwendung von 2-Propargyloxy-benzazolen als Synergisten in Schädlingsbekämpfungsmitteln.

Insektizide und akarizide Wirkstoffe bzw. Wirkstoffgruppen der verschiedensten Art sind bereits bekannt, z.B.:

A) Carbaminsäureester, wie z.B. N-Methyl-O-(2-iso-propoxyphenyl)-carbaminsäureester (Propoxur), N-Methyl-O-(2,3-dihydro-2,2-dimethyl-7-benzofuranyl)-carbaminsäureester (Carbofuran), N-Methyl-O-(2,3-dimethyl-methylendioxy-phenyl)-carbaminsäureester (Bendiocarb) und N-Methyl-O-(1-methyl-thio-ethyliden-amino)-carbaminsäureester (Methomyl),

B) Carbonsäureester, wie z.B. die natürlich vorkommenden Pyrethroide sowie synthetische Pyrethroide, wie z.B. 3-(2-Methyl-1-propenyl)-2,2-dimethyl-cyclopropancarbonsäure-3,4,5,6-tetrahydro-phthalimidomethylester (Tetramethrin), 3-(2,2-Dichlor-vinyl)-2,2-dimethyl-cyclopropancarbonsäure-3-phenoxybenzylester (Permethrin), 1-R-cis-3-(2,2-Dibromvinyl)-2,2-dimethyl-cyclopropan-carbonsäure-α-S-cyano-3-phenoxy-benzylester (Decamethrin) und 3-(2,2-Dichlorvinyl)-2,2-dimethyl-cyclopropancarbonsäure-pentafluorbenzylester sowie weitere Carbonsäureester wie z.B. Essigsäure-(1-(3,4-dichlorphenyl)-2,2,2-trichlorethyl-ester (Penfenate),

C) Phosphorsäureester, wie z.B. O,O-Dimethyl-O-(2,2-dichlor-vinyl)-phosphorsäureester (DDVP) und O,O-Dimethyl-S-(N-methyl-aminocarbonylmethyl)-thiolphosphorsäureester (Omethoate), und

D) Halogenkohlenwasserstoffe, wie z.B. γ-1,2,3,4,5,6-Hexachlorcyclohexan (Lindane), DDT und Methoxychlor.

Weiter sind synergistische Mischungen von Carbaminsäureestern, wie z.B. N-Methyl-O-(2-iso-propoxyphenyl)-carbaminsäureester, oder von Phosphorsäureestern, z.B. O,O-Diethyl-O-(2-isopropyl-4-methylpyrimidin(6)yl)-thionophosphorsäureester, oder von natürlichen oder synthetischen Pyrethroiden mit Piperonylethern, wie z.B. α-(2-(2-Butoxy-ethoxy)-ethoxy)-4,5-methylendioxy-2-propyltoluol, bekannt (vgl. Bul. Org. Mond. Santé/Bull. Wld. Hlth Org. 1966, 35, 691-708; Schrader, G.: Die Entwicklung neuer insektizider Phosphorsäureester 1963, S. 158). Doch ist die Wirksamkeit dieser synergistischen Wirkstoffkombinationen nicht befriedigend. Eine gewisse praktische Bedeutung hat bisher nur das α-(2-(2-Butoxyethoxy)-ethoxy)-4,5-methylendioxy-2-propyl-toluol erlangt. In der US-A- 2 769 010 wird 2-(2-Propinoxy)-benzthiazol als Herbizid beschrieben. Aus JP-A- 8 038 302 ist 2-(2-Propinoxy)-benzoxazol bekannt. Bestimmte 2-(2-Propinoxy)-benzazole sind aus JP-A- 7 920 137 als Akarizide bekannt.

Es wurde nun gefunden, dass die 2-Propargyloxy-benzazole der Formel I

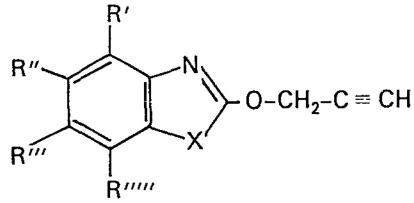

(I)

in welcher

R′, R″, R‴ und R⁗ unabhängig voneinander für Wasserstoff, Halogen oder $C_1$-$C_4$-Alkyl stehen und

X für Sauerstoff oder Schwefel steht,

zusammen mit anderen Wirkstoffen, welche gegen Arthropoden wirksam sind,

wobei diese Wirkstoffe einzeln oder in Mischung vorliegen können,

eine besonders hohe Wirkung gegen Arthropoden, vorzugsweise gegen Insekten, Milben und Spinnentiere, insbesondere gegen Insekten aufweisen, und somit als Synergisten in Schädlingsbekämpfungsmitteln verwendet werden können.

Bevorzugt werden hierbei Wirkstoffkombinationen aus 2-Propargyloxybenzazolen der Formel (I) und

A) Carbaminsäureestern und/oder

B) Carbonsäureestern einschliesslich der natürlichen sowie synthetischen Pyrethroide und/oder

C) Phosphorverbindungen, insbesondere Phosphorsäure- und Phosphonsäureestern und/oder

D) Halogenkohlenwasserstoffen verwendet.

Die vorliegende Erfindung betrifft somit die Verwendung von synergistisch wirkenden 2-Propargyloxy-benzazolen der Formel (I) als Synergisten in Schädlingsbekämpfungsmitteln, vorzugsweise zur Bekämpfung von Insekten, Milben und Spinnentieren, insbesondere von Insekten.

Die synergistische Wirkung der Verbindungen der allgemeinen Formel (I) erstreckt sich praktisch auf alle gegen Arthropoden wirksamen Verbindungsklassen. Sie ist bevorzugt bei den folgenden Wirkstoffen von Interesse:

A) Carbaminsäureestern der Formel II

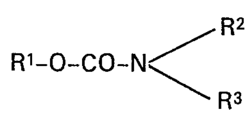

(II)

in welcher

$R^1$ für einen gegebenenfalls substituierten carbocyclischen oder heterocyclischen aromatischen Rest oder für einen gegebenenfalls substituierten Oximrest steht,

$R^2$ für $C_1$-$C_4$-Alkyl steht und

$R^3$ für Wasserstoff, $C_1$-$C_4$-Alkyl oder für den Rest Z steht, wobei

Z für den Rest -CO-$R^4$ steht, worin

$R^4$ für Halogen, $C_1$-$C_4$-Alkyl, $C_1$-$C_4$-Alkoxy, $C_3$-$C_5$-Alkenoxy, $C_3$-$C_5$-Alkonoxy, $C_1$-$C_4$-Alkylthio, $C_1$-$C_4$-Alkylamino, Di-$C_1$-$C_4$-Alkyl-amino, $C_1$-$C_4$-Alkylhy-

droxylamino, für gegebenenfalls durch Halogen, Nitro, Cyano, Trifluormethyl, $C_1$-$C_4$-Alkyl, $C_1$-$C_4$-Alkoxy, $C_1$-$C_4$-Alkylendioxy, $C_1$-$C_4$-Alkylthio, $C_1$-$C_4$-Alkoxy-carbonyl substituiertes Phenoxy, Phenylthio oder Phenylamino, für 2,3-Dihydroxy-2,2-dimethyl-7-benzofuranyl oder für den Rest

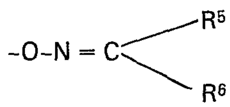

steht, worin

$R^5$ für Wasserstoff, $C_1$-$C_4$-Alkyl oder Di-$C_1$-$C_4$-alkyl-amino-carbonyl steht und

$R^6$ für $C_1$-$C_4$-Alkyl, $C_1$-$C_4$-Alkylthio, Cyano-$C_1$-$C_4$-alkylthio, $C_1$-$C_4$-Alkylthio-$C_1$-$C_4$-alkyl steht,

oder die beiden Reste $R^5$ und $R^6$ zusammen für gegebenenfalls durch Sauerstoff, Schwefel, SO oder $SO_2$ unterbrochenes $C_2$-$C_8$-Alkandiyl stehen, in welcher weiter

Z für den Rest $-S_n(O)_m-R^7$ steht, worin

n für 1 oder 2 und

m für Null, 1 oder 2 stehen und

$R^7$ für gegebenenfalls durch Halogen substituiertes $C_1$-$C_4$-Alkyl, $C_3$-$C_5$-Alkenyl, $C_3$-$C_5$-Alkinyl oder $C_3$-$C_6$-Cycloalkyl, für gegebenenfalls durch Halogen, Cyano, Nitro, Trifluormethyl, Trifluormethoxy, Trifluormethylthio, $C_1$-$C_4$-Alkyl oder $C_1$-$C_4$-Alkoxy substituiertes Phenyl, Benzyl oder Phenylethyl oder für den Rest

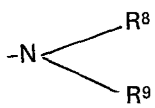

steht, worin

$R^8$ für $C_1$-$C_4$-Alkyl, $C_3$-$C_5$-Alkenyl, $C_3$-$C_5$-Alkinyl, $C_3$-$C_6$-Cycloalkyl oder Benzyl steht und

$R^9$ für $C_1$-$C_4$-Alkyl, $C_3$-$C_5$-Alkenyl, $C_3$-$C_5$-Alkinyl, $C_3$-$C_6$-Cycloalkyl, Benzyl, Phenylethyl, Halogencarbonyl, Formyl, $C_1$-$C_4$-Alkyl-carbonyl, $C_1$-$C_4$-Alkoxycarbonyl, $C_1$-$C_4$-Alkoxy-phenoxy-carbonyl, $C_3$-$C_5$-Alkinoxy-carbonyl, $C_3$-$C_5$-Alkenoxy-carbonyl, $C_1$-$C_5$-Alkylthiocarbonyl, $C_1$-$C_4$-Alkyl-amino-carbonyl, $C_1$-$C_4$-Alkyl-hydroxylamino-carbonyl, $C_1$-$C_{10}$-Alkylphenoxycarbonyl, Di-$C_1$-$C_4$-alkyl-amino-carbonyl, Phenylthiocarbonyl, Phenoxycarbonyl, 2,3-Dihydro-2,2-Dimethyl-7-benzofuranyl-oxycarbonyl, für gegebenenfalls durch Halogen, Cyano, Nitro, Trifluormethyl, $C_1$-$C_{10}$-Alkyl oder $C_1$-$C_4$-Alkoxy substituiertes Phenylsulfenyl, Phenylsulfinyl, Phenylsulfonyl oder Phenyl steht, oder für den Rest

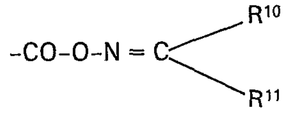

steht, worin

$R^{10}$ die oben für $R^5$ angegebene und

$R^{11}$ die oben für $R^6$ angegebene Bedeutung hat, wobei ferner im Rest

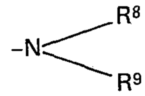

die Reste $R^8$ und $R^9$ zusammen für eine gegebenenfalls durch Sauerstoff oder Schwefel unterbrochene Kohlenwasserstoffkette mit 3 bis 8 Kohlenstoffatome stehen und worin weiter $R^7$ für den gleichen Rest stehen kann an den der Rest $-S_n(O)_m-R^7$ gebunden ist.

Als Wirkstoffkomponenten ganz besonders bevorzugt sind Carbaminsäureester der Formel (II), in welcher

$R^1$ für gegebenenfalls durch $C_1$-$C_4$-Alkyl, $C_2$-$C_4$-Alkenyl, $C_1$-$C_4$-Alkoxy, $C_1$-$C_4$-Alkoxy-methyl, $C_1$-$C_4$-Alkylthio, $C_1$-$C_4$-Alkylthio-methyl, $C_1$-$C_4$-Alkylamino, Di-($C_1$-$C_4$-alkyl)-amino, Di-($C_3$-$C_4$-Alkenyl)-amino, Halogen, Dioxolanyl, Methylendioxy und/oder durch den Rest $-N{=}CH-N(CH_3)_2$ substituierte Reste aus der Reihe Phenyl, Naphthyl, 2,3-Dihydro-7-benzofuranyl, Pyrazolyl oder Pyrimidinyl steht, oder in welcher

$R^1$ für einen Alkylidenaminorest der Formel IIa

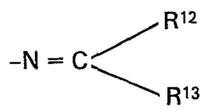

(IIa)

steht, in welcher

$R^{12}$ und $R^{13}$ die oben für $R^5$ bzw. $R^6$ angegebenen Bedeutung haben.

Als Beispiele für die Carbaminsäureester der Formel (II) seien genannt:

2-Methyl-phenyl-, 2-Ethyl-phenyl-, 2-iso-Propylphenyl-, 2-sek.-Butyl-phenyl-, 2-Methoxy-phenyl-, 2-Ethoxy-phenyl-, 2-iso-Propoxy-phenyl-, 4-Methyl-phenyl-, 4-Ethyl-phenyl-, 4-n-Propylphenyl-, 4-Methoxy-phenyl-, 4-Ethoxy-phenyl-, 4-n-Propoxy-phenyl-, 3,4,5-Trimethyl-phenyl-, 3,5-Dimethyl-4-methylthio-phenyl-, 3-Methyl-4-dimethylamino-phenyl-, 2-Ethylthiomethyl-phenyl-, 1-Naphthyl-, 2,3-Dihydro-2,2-dimethyl-7-benzofuranyl-, 2,3-(Dimethyl-methylendioxy)-phenyl-, 2-(4,5-Dimethyl-1,3-dioxolan-2-yl)-phenyl-, 1-Methylthio-ethyliden-amino-, 2-Methylthio-2-methyl-propyliden-amino-, 1-(2-Cyano-ethylthio)-ethyliden-amino- und 1-Methylthiomethyl-2,2-dimethyl-propyliden-amino-N-methyl-carbaminsäureester, wobei 2-iso-Propoxy-phenyl-N-methyl-carbaminsäureester als besonders bevorzugte Verbindung genannt sei.

Die synergistische Wirkung der Verbindung der Formel (I) zeigt sich weiter vorzugsweise bei

B) Carbonsäureestern der Formel III

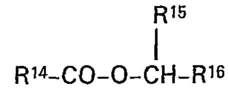

in welcher

$R^{14}$ für einen offenkettigen oder cyclischen Alkylrest steht, der gegebenenfalls substituiert

ist durch Halogen-, Alkyl, Cycloalkyl, durch gegebenenfalls durch Halogen und/oder Alkoxy substituiertes Alkenyl, durch Phenyl oder Styryl, welche gegebenenfalls durch Halogen, gegebenenfalls halogen-substituierte Reste der Reihe Alkyl, Alkoxy, Alkylendioxy und/oder Alkylthio substituiert sind, durch spirocyclisch verknüpftes, gegebenenfalls halogensubstituiertes Cycloalk(en)yl, welches gegebenenfalls benzannelliert ist, in welcher weiter

$R^{15}$ für Wasserstoff, Alkyl, Halogenalkyl, Alkenyl, Alkinyl, oder Cyano steht, und

$R^{16}$ für einen gegebenenfalls substituierten Alkyl- oder Arylrest oder für einen Heterocyclus steht, oder zusammen mit $R^{15}$ und dem Kohlenstoffatom, an das beide Reste gebunden sind, einen Cyclopentenonring bildet.

Ganz besonders als Wirkstoffkomponenten bevorzugt sind Carbonsäureester der Formel (III), in welcher

$R^{14}$ für den Rest

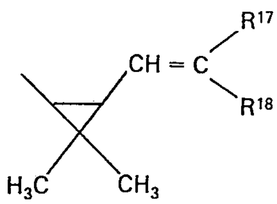

steht, worin

$R^{17}$ für Wasserstoff, Methyl, Fluor, Chlor oder Brom und

$R^{18}$ für Methyl, Fluor, Chlor, Brom, $C_1$-$C_2$-Fluoralkyl oder $C_1$-$C_2$-Chlorfluoralkyl oder für gegebenenfalls durch Halogen und/oder gegebenenfalls halogen-substituierte Reste der Reihe $C_1$-$C_4$-Alkyl, $C_1$-$C_4$-Alkoxy, $C_1$-$C_4$-Alkylthio und/oder $C_1$-$C_2$-Alkylendioxy substituiertes Phenyl steht und worin beide Reste $R^{17}$ und $R^{18}$ für $C_2$-$C_5$-Alkandiyl (Alkylen) stehen; oder in welcher

$R^{14}$ für den Rest

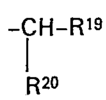

steht, worin

$R^{19}$ für gegebenenfalls durch Halogen und/oder durch gegebenenfalls halogen-substituierte Reste der Reihe $C_1$-$C_4$-Alkyl, $C_1$-$C_4$-Alkoxy, $C_1$-$C_4$-Alkylthio oder $C_1$-$C_2$-Alkylendioxy substituiertes Phenyl steht und

$R^{20}$ für Isopropyl oder Cyclopropyl steht;

oder in welcher

$R^{14}$ für einen der Reste

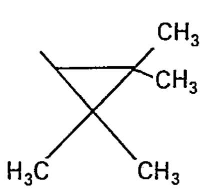

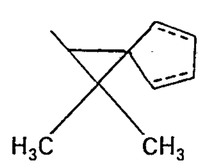

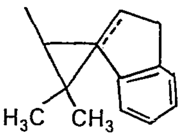

oder Methyl

steht, in welcher weiter

$R^{15}$ für Wasserstoff, $C_1$-$C_4$-Alkyl, $C_1$-$C_4$-Halogenalkyl, Cyano oder Ethinyl steht und

$R^{16}$ für gegebenenfalls durch Halogen und/oder durch einen gegebenenfalls halogen-substituierten Rest der Reihe $C_1$-$C_4$-Alkyl, $C_2$-$C_4$-Alkenyl, $C_1$-$C_4$-Alkoxy, $C_2$-$C_4$-Alkenoxy, $C_1$-$C_4$-Alkylthio, $C_1$-$C_2$-Alkylendioxy, Phenoxy und/oder Benzyl-substituierte Reste Phenyl, Furyl oder Tetrahydrophthalimido steht.

Weiter sind die natürlich vorkommenden Pyrethroide besonders bevorzugt.

Als Beispiele für die Carbonsäureester der Formel (III) seien genannt:

Essigsäure-(2,2,2-Trichlor-1-(3,4-dichlorphenyl)-ethyl)-ester, 2,2-Dimethyl-3-(2-methyl-propen-1-yl)-cyclopropancarbonsäure-(3,4,5,6-tetrahydrophthalimido-methyl)-ester, 2,2-Dimethyl-3-(2,2-dichlor-vinyl)-cyclopropancarbonsäure-(3-phenoxy-benzyl)-ester, 2,2-Dimethyl-3-(2,2-dichlor-vinyl)-cyclopropan-carbonsäure-(α-cyano-4-fluor-3-phenoxy-benzyl)-ester, 2,2-Dimethyl-3-(2,2-dichlorvinyl)-cyclopropancarbonsäure-(pentafluor-benzyl)-ester, 2,2-Dimethyl-3-(2,2-dibrom-vinyl)-cyclopropan-carbonsäure-(α-cyano-3-phenoxy-benzyl)-ester, 3-Methyl-2-(4-chlor-phenyl)-butansäure-(α-cyano-3-phenoxy-benzyl)-ester und 2,2-Dimethyl-3-(2,2-dichlor-vinyl)-cyclopropancarbonsäure-(α-cyano-3-phenoxy-benzyl)-ester.

Weiterhin zeigt sich die synergistische Wirkung der Verbindungen der allgemeinen Formel (I) bevorzugt bei

C) Phosphorverbindungen, insbesondere Phosphorsäure- und Phosphonsäureestern der allgemeinen Formel IV

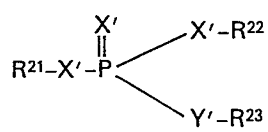

(IV)

in welcher

X′ jeweils für O oder S steht und

Y′ für O, S, -NH- oder für eine direkte Bindung zwischen dem zentralen P-Atom und dem $R^{23}$ steht und

$R^{21}$ und $R^{22}$ gleich oder verschieden sind und für gegebenenfalls substituiertes Alkyl oder Aryl stehen

$R^{23}$ für Wasserstoff, gegebenenfalls substituiertes Alkyl, Aryl, Heteroaryl, Aralkyl, Alkenyl, Dioxanyl oder einen Oximrest oder für den gleichen Rest steht, an den es gebunden ist.

Besonders bevorzugt sind Phosphorsäure- und Phosphonsäureester der Formel (IV), in welcher

$R^{21}$ und $R^{22}$ gleich oder verschieden sind und für $C_1$-$C_4$-Alkyl oder Phenyl stehen,

$R^{23}$ für Alkyl mit 1 bis 4 Kohlenstoffatomen steht, das gegebenenfalls durch Halogen, Hydroxyl, Cyano, gegebenenfalls halogen-substituiertes Phenyl, Carbamoyl, Alkylsulfonyl, Alkylsulfinyl, Alkylcarbonyl, Alkoxy, Alkylmercapto, Alkoxycarbonyl, Alkylaminocarbonyl, letztere mit jeweils bis zu 6 Kohlenstoffatomen, substituiert ist, für Alkenyl mit bis zu 4 Kohlenstoffatomen, das gegebenenfalls durch Halogen, gegebenenfalls halogensubstituiertes Phenyl oder $C_1$-$C_4$-Alkoxycarbonyl substituiert ist, oder für den Rest der allgemeinen Formel IV a

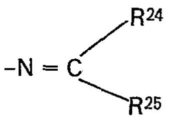

(IVa)

wobei $R^{24}$ und $R^{25}$ die oben für $R^5$ bzw. $R^6$ angegebene Bedeutung besitzen, oder für Cyano oder Phenyl stehen,

$R^{23}$ steht ferner für Dioxanyl, das durch denselben Rest substituiert ist, an den $R^{23}$ gebunden ist, oder $R^{23}$ steht für den gleichen Rest, an den es gebunden ist, oder $R^{23}$ steht für Phenyl, das gegebenenfalls durch Methyl, Nitro, Cyano, Halogen und/oder Methylthio substituiert ist; $R^{23}$ steht ausserdem besonders bevorzugt für gegebenenfalls durch $C_1$-$C_4$-Alkoxy, $C_1$-$C_4$-Alkylthiomethyl, $C_1$-$C_4$-Alkyl und/oder Halogen substituierte heteroaromatische Reste, wie Pyridinyl, Chinolinyl, Chinoxalinyl, Pyrimidinyl oder Benzo-1,2,4-triazinyl.

Im einzelnen seien beispielhaft genannt:
O,O-Dimethyl- bzw. O,O-Diethyl-O-(2,2-dichlor- bzw. 2,2-dibromvinyl)-phosphorsäureester,
O,O-Diethyl-O-(4-nitro-phenyl)-thionophosphorsäureester, O,O-Dimethyl-O-(3-methyl-4-methylthio-phenyl)-thiono-phosphorsäureester,
O,O-Dimethyl-O-(3-methyl-4-nitro-phenyl)-thionophosphorsäureester,
O-Ethyl-S-n-propyl-O-(2,4-dichlor-phenyl)-thionophosphorsäureester,
O-Ethyl-S-n-propyl-O-(4-methylthio-phenyl)-thionophosphorsäureester,
O,O-Dimethyl-S-(4-oxo-1,2,3-benzotriazin(3)yl-methyl)-thionothiolphosphorsäureester,
O-Methyl-O-(2-iso-propyl-6-methoxy-pyrimidin(4)yl)-thionomethanphosphonsäureester,
O,O-Diethyl-O-(2-iso-propyl-6-methyl-pyrimidin(4)yl)-thionophosphorsäureester,
O,O-Diethyl-O-(3-chlor-4-methyl-cumarin(7(yl)-thionophosphorsäureester,
O,O-Dimethyl-2,2,2-trichlor-1-hydroxy-ethanphosphonsäureester,
O,O-Dimethyl-S-(methylaminocarbonyl-methyl)-thionophosphorsäureester.

Die synergistische Wirkung der Verbindungen der Formel (I) zeigt sich ferner vorzugsweise bei D) Halogenkohlenwasserstoffen, wie z.B. γ-1,2,3,4,5,6-Hexachlorcyclohexan und 1,1,1-Trichlor-2,2-bis-(4-chlor-phenyl)-ethan.

Einen guten Überblick über erfindungsgemäss verwendbare Carbamate, Carbonsäureester und Phosphorverbindungen liefert auch «Pflanzenschutz und Schädlingsbekämpfung», herausgegeben von K.H. Büchel, Thieme Verlag Stuttgart, 1977.

Die erfindungsgemäss als Synergisten zu verwendenden 2-Propargyloxybenzazole sind durch Formel (I) definiert.

Alkyl, R′ bis R″″ enthält 1 bis 4, insbesondere 1 oder 2 Kohlenstoffatome, wobei Methyl, Ethyl, n- und i-Propyl und n-, i-, sec.- und t-Butyl genannt seien.

Halogen als R′ bis R″″ steht für Fluor, Chlor, Jod und Brom, vorzugsweise für Chlor und Brom, insbesondere für Chlor.

Insbesondere stehen in Formel (I) R′, R″, R‴ und R″″ unabhängig voneinander für Wasserstoff, Chlor oder Methyl und X für Sauerstoff oder Schwefel.

Als Beispiele für die Verbindungen der Formel (I) seien genannt:
2-Propargyloxy-benzoxazol und 2-Propargyloxybenzthiazol, 5-Chlor-2-propargyloxy-benzoxazol und 5-Chlor-2-propargyloxy-benzthiazol, 6-Chlor-2-propargyloxy-benzoxazol und 6-Chlor-2-propargyloxy-benzthiazol, 5,7-Dichlor-2-propargyloxy-benzoxazol und 5,7-Dichlor-2-propargyloxy-benzthiazol, 5-Methyl-2-propargyloxy-benzoxazol und 5-Methyl-2-propargyloxy-benzthiazol.

Wo in der Beschreibung nicht anders angegeben, enthalten Alkylreste oder die Alkylteile von Resten, welche Alkylgruppen enthalten, vorzugsweise 1 bis 6, insbesondere 1 bis 4 Kohlenstoffatome und sind geradkettig oder verzweigt, wobei beispielhaft Methyl, Ethyl, n- und i-Propyl genannt seien.

Alkenyl- und Alkinylgruppen enthalten vorzugsweise 2 bis 6, insbesondere 2 bis 4 Kohlenstoffatome und vorzugsweise 1 oder 2, insbesondere 1 Doppel- bzw. Dreifachbindungen.

Arylgruppen sind vorzugsweise Naphthyl und Phenyl, insbesondere Phenyl.

Heterocyclische Gruppen enthalten vorzugsweise 5 bis 7, insbesondere 5 oder 6 Ringglieder und können ein oder mehrere Heteroatome, vorzugsweise 1 bis 3, insbesondere 1 oder 2 Heteroatome, wie Stickstoff, Sauerstoff und/oder Schwefel enthalten.

Halogen steht für Fluor, Chlor, Brom und Jod, vorzugsweise für Fluor, Chlor und Brom, insbesondere für Chlor und Fluor.

Mehrfach substituierte Reste enthalten vorzugsweise 1 bis 5, insbesondere 1 bis 3 gleiche oder verschiedene Substituenten.

Die Verbindungen der Formel (I) können nach in der Literatur beschriebenen Verfahren in bekannter Weise hergestellt werden (vgl. Japanische Patentanmeldung 54 020 137).

Die hierfür einzusetzenden 2-Halogen-benzazole sind bekannt und/oder nach bekannten Verfahren erhältlich (vgl. Deutsche Offenlegungsschrift 1 670 706, Deutsche Auslegeschrift 1 210 617 und Britische Patentschrift 966 496).

Man erhält die Verbindungen der Formel (I) dadurch, dass man 2-Halogenbenzazole der Formel (V)

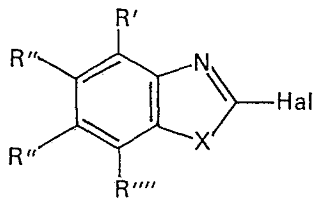

(V)

in welcher
Hal für Halogen steht und X und R′ bis R″″ die oben angegebene Bedeutung haben, mit Propargylalkohol in Gegenwart einer Base und gegebenenfalls in Gegenwart eines Lösungsmittels umsetzt.

Als Verdünnungsmittel kommen alle inerten organischen Lösungsmittel in Frage. Hierzu gehören vorzugsweise aliphatische und aromatische Kohlenwasserstoffe, wie Pentan, Hexan, Cyclohexan, Benzol, Toluol und Xylol; Ether, wie Diethylether, Dibutylether, Glykoldimethylether, Tetrahydrofuran und Dioxan; Amide, wie Dimethylformamid und Alkohole, wie Methanol, Ethanol und i-Propanol sowie überschüssiger Propargylalkohol.

Als Säurebindemittel kommen alle üblichen Säurebindemittel in Frage. Hierzu gehören vorzugsweise Alkali- und Erdalkalimetallhydroxide, wie KOH, NaOH, Mg- und Ca-Hydroxid; Alkalicarbonate, wie $Na_2CO_3$ und $K_2CO_3$; Alkalialkoholate, wie Natriummethylat, Kaliumethylat und Kalium-t-butylat; Alkalimetalle, deren Amide und Hydride.

Die Reaktionstemperaturen können in einem grösseren Bereich variiert werden. Im allgemeinen arbeitet man zwischen etwa −20 und etwa 100°C, vorzugsweise zwischen etwa 0° und 80°C.

Bei der Durchführung des Verfahrens setzt man auf 1 Mol 2-Halogenbenzazol der Formel (V) vorzugsweise 1 bis 1,5 Mol, insbesondere 1,1 bis 1,2 Mol bzw. Äquivalente des Säurebindemittels ein.

Je Mol 2-Halogenbenzazol der Formel (V) wird vorzugsweise wenigstens 1 Mol Propargylalkohol eingesetzt. Propargylalkohol kann auch ohne Nachteil in einem grösseren Überschuss verwendet werden.

Die Reaktionstemperaturen werden zweckmässigerweise bei niederen Temperaturen zwischen ca. −20 bis +20°C zusammengegeben und anschliessend unter Rühren langsam erwärmt.

Die Aufarbeitung und Isolierung der Produkte kann nach üblichen Methoden erfolgen, beispielsweise durch Schütteln mit Wasser und einem mit Wasser praktisch nicht mischbaren organischen Lösungsmittel, wie z.B. Methylenchlorid, Abtrennen der organischen Phase, Waschen, mit Wasser, Trocknen, Filtrieren und Einengen bzw. völligem Destillieren des Filtrats.

Wie bereits erwähnt, zeigen die erfindungsgemäss verwendbaren 2-Propargyloxybenzole der Formel (I) mit anderen Wirkstoffen, wie Carbaminsäureestern, Carbonsäureestern, Phosphor- und Phosphonsäureestern und halogenierten Kohlenwasserstoffen eine hervorragende Wirkungssteigerung gegenüber den Einzelwirkstoffen bzw. gegenüber deren Summe.

Überraschenderweise ist die pestizide Wirkung dieser Wirkstoffkombinationen wesentlich höher als die Wirkung der Einzelkomponenten bzw. die Summe der Wirkungen der Einzelkomponenten. Sie ist ferner wesentlich höher als die Wirkung von Wirkstoffkombinationen mit dem bekannten Synergisten Piperonylbutoxid. Ausserdem zeigen die erfindungsgemäss verwendbaren 2-Propargyloxy-benzazole ausgezeichnete synergistische Wirksamkeit nicht nur bei einer Wirkstoffklasse, sondern bei den Wirkstoffen aus den verschiedensten chemischen Stoffgruppen.

Die Gewichtsverhältnisse der Synergisten und Wirkstoffe können in einem relativ grossen Bereich variiert werden. Im allgemeinen werden die als Synergisten verwendeten Verbindungen der Formel (I) mit den übrigen Wirkstoffen im Verhältnis 0,1:10 bis 10:0,1, vorzugsweise 0,2 bis 5 bis 5 bis 0,2, insbesondere 0,5:1,0 bis 1,0:1,0 eingesetzt.

Durch die Verwendung der Verbindungen der Formel (I) als Synergisten erreicht man nicht nur eine schnelle knock-down-Wirkung, sondern auch die nachhaltige Abtötung der tierischen Schädlinge, vorzugsweise in Insekten und Milben, insbesondere von Insekten, die in der Landwirtschaft, in Forsten, im Vorrats- und Materialschutz sowie im Hygienebereich vorkommen, wobei normal sensible und resistente Arten sowie gegen alle oder einzelne Entwicklungsstadien erfasst werden.

Zu den oben erwähnten Schädlingen gehören:

Aus der Ordnung der Isopoda z.B. Oniscus asellus, Porcellio scaber.

Aus der Ordnung der Thysanura z.B. Lepisma saccharina.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Pediculus humanus corporis, Haematopinus spp., Linograthus spp.

Aus der Ordnung der Mallophaga z.B. Trichodectes spp., Damalinea spp.

Aus der Ordnung der Heteroptera z.B. Cimex lectularius, Rhodnius prolixus, Triatoma spp.

Aus der Ordnung der Homoptera z.B. Myzus spp., und Psylla spp.

Aus der Ordnung der Lepidoptera z.B. Ephestia kuehniella und Galleria mellonella.

Aus der Ordnung der Coleptera z.B. Anobium punctatum, Rhizopertha dominica, Bruchidius obtectus, Acanthoscelides obtectus, Hylotrupes bejulus, Oryzaephilus surinamensis, Sitrophilus spp., Dermestes spp., Trogoderma spp., Anthrenus spp., Attagenus spp., Lyctus spp., Ptinus

spp., Niptus hololeucus, Gibbium psylloides, Tribolium spp.. und Tenebrio molitor.

Aus der Ordnung der Hymenoptera z.B. Lasius spp., Monomorium pharaonis, Vespa spp.

Aus der Ordnung der Diptera z.B. Aëdes spp., Anopheles spp., Culex spp., Drosophila melanogaster, Musca spp., Fannia spp., Calliphora erythrocephala, Lucilia spp., Chrysomyia spp., Gastrophilus spp., Hyppobosca spp., Stomoxys spp., Oestrus spp., Hypoderma spp. und Tabanus spp.

Aus der Ordnung der Siphonaptera z.B. Yenopsylla cheopis, Ceratophyllus spp.

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Boophilus spp., Rhipicephalus spp., Amblyomma spp., Hyalomma spp., Ixodes spp., Psoroptes spp., Chorioptes spp., Sarcoptes spp.

Die Wirkstoffkombinationen aus den erfindungsgemäss verwendbaren Verbindungen der Formel (I) und den anderen Wirkstoffen können in die üblichen Formulierungen übergeführt werden, wie Lösungen, Emulsionen, Spritzpulver, Suspensionen, Pulver, Stäubemittel, Schäume, Pasten, lösliche Pulver, Aerosole, Suspensions-Emulsionskonzentrate, Wirkstoff-imprägnierte Natur- und synthetische Stoffe, Feinstverkapselungen in polymeren Stoffen, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssiger Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphtaline, chlorierte Aromaten oder chlorierte alophatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol, sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnussschalen, Maiskolben und Tabakstengel; als Emulgier- und/oder schaumerzeugende Mittel: nichtionogene und anionische Emulgatoren, wie Polyoxyethylen-Fettsäure-Ester, Polyoxyethylen-Fettalkohol-Ether, z.B. Alkylaryl-polyglykol-ether, Alkylsulfonate; als Dispergiermittel: z.B. Lignin, Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe, und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoffkombination, vorzugsweise zwischen 0,5 und 90%.

Die Anwendung der Wirkstoffkombinationen erfolgt in Form ihrer handelsüblichen Formulierungen und/oder den aus diesen Formulierungen bereiteten Anwendungsformen.

Der gesamte Wirkstoffgehalt der aus den handelsüblichen Formulierungen bereiteten Anwendungsformen kann in weiten Bereichen variieren. Die Wirkstoffkonzentration der Anwendungsformen kann von 0,0001 bis zu 100 Gew.-% Wirkstoffkombination, vorzugsweise zwischen 0,01 und 10 Gew.-% liegen.

Die Anwendung geschieht in einer den Anwendungsformen angepassten üblichen Weise.

Bei der Anwendung gegen Hygiene- und Vorratsschädlinge zeichnen sich die Wirkstoffkombinationen durch eine hervorragende Residualwirkung auf Holz und Ton sowie durch eine gute Alkalistabilität auf gekälkten Unterlagen aus.

Die folgenden Beispiele zeigen Wirkstoffe und erfindungsgemäss verwendbare 2-Propargyloxybenzazole, anhand deren Kombination beispielshaft eine synergistische Wirkung erläutert wird:

I. Beispiele für verwendbare «andere» Wirkstoffe

A) 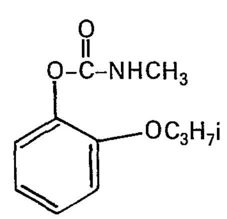 (Propoxur)

B) 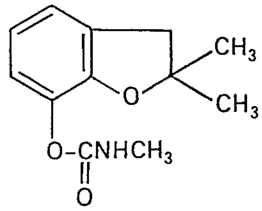 (Carbofuran)

C) 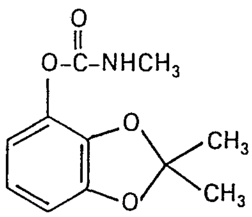 (Bendiocarb)

D) 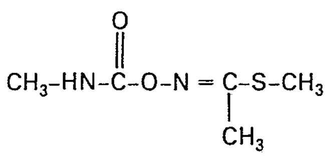 (Methomyl)

E) Pyrethine natürlicher Herkunft als 25%iger Extrakt

F) 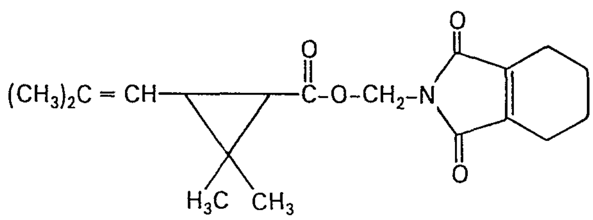 (Tetramethrin)

G) 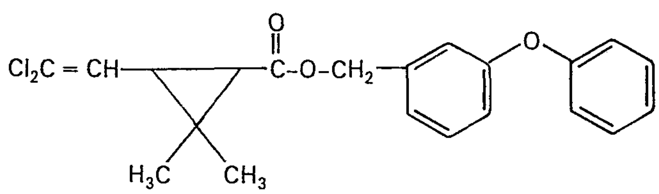 (Permethrin)

H) 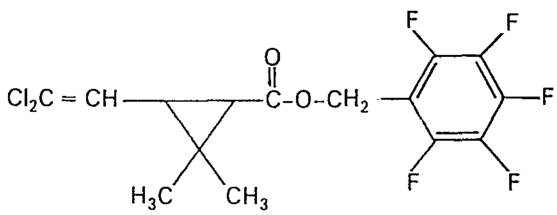

I) 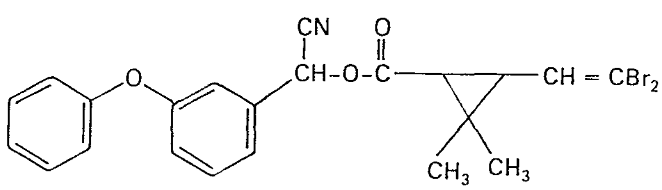 (Decamethrin)

J) 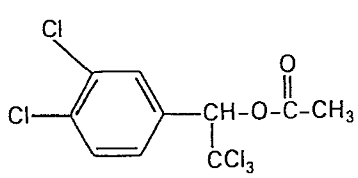 (Penfenate)

K) 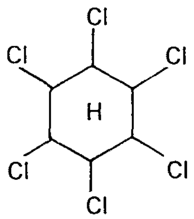 (γ-Hexachlorcyclohexan)

L) 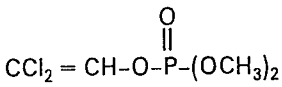 (DDVP)

M) 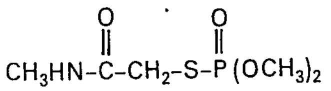 (Omethoat)

II. Beispiele von erfindungsgemäss verwendbaren Synergisten

1) 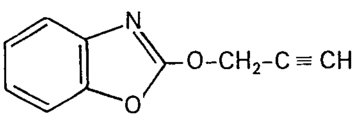

2) 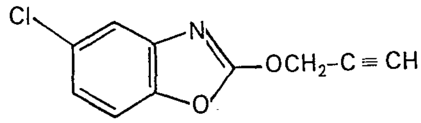

3) 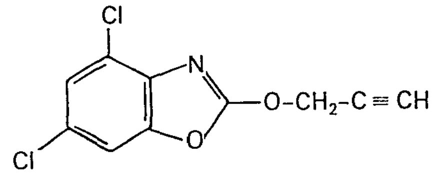

4) 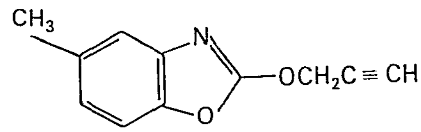

5) 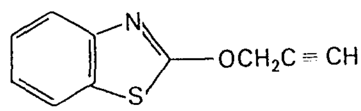

Standard-Synergist:
6) Piperonylbutoxid (bekannt)

| Wirkstoff Kennbuchstabe | + | Synergist-Nr. | Konzentrationen in % Wirkstoff | + | Synergist | $LT_{100}$ nach Minuten, bzw. % nach Minuten |
|---|---|---|---|---|---|---|
| A) | | – | 1,0 | | – | 360′ = 0 % |
| B) | | – | 1,0 | | – | 360′ = 70 % |
| C) | | – | 1,0 | | – | 360′ = 35 % |
| D) | | – | 1,0 | | – | 360′ = 80 % |
| E) | | – | 0,2 | | – | 90′ |
| F) | | – | 0,04 | | – | 360′ = 90 % |
| G) | | – | 0,04 | | – | 105′ |
| H) | | – | 0,0016 | | – | 60′ |
| I) | | – | 0,0016 | | – | 105′ |
| J) | | – | 1,0 | | – | 360′ = 70 % |
| K) | | – | 0,04 | | – | 210′ |
| L) | | – | 0,008 | | – | 150′ |
| M) | | – | 0,04 | | – | 360′ = 45 % |
| – | | 1) | – | | 0,04 | 360′ = 60 % |
| – | | 2) | – | | 1,0 | 360′ = 90 % |
| – | | 3) | – | | 1,0 | 360′ = 5 % |
| – | | 4) | – | | 1,0 | 360′ = 0 % |
| – | | 5) | – | | 0,04 | 360′ = 60 % |
| – | | 6) (Standard) | – | | 1,0 | 360′ = 0 % |
| A | + | 6 | 1,0 | + | 1,0 | 360′ |
| A | + | 1 | 0,008 | + | 0,008 | 150′ |
| A | + | 2 | 0,04 | + | 0,04 | 120′ |
| A | + | 3 | 0,04 | + | 0,04 | 120′ |
| A | + | 4 | 0,04 | + | 0,04 | 210′ |
| A | + | 5 | 0,04 | + | 0,04 | 90′ |

Versuchsbeispiele

$LT_{100}$-Test

Testtiere: Musca domestica, Stamm Weymanns, (gegen Carbaminsäureester und p-Ester resistent)

Lösungsmittel: Aceton

Von den Wirkstoffen, Synergisten und Gemischen aus Wirkstoffen und Synergisten werden Lösungen hergestellt und 2,5 ml davon in Petrischalen auf Filterpapier von 9,5 cm Durchmesser

pipettiert. Das Filterpapier saugt die Lösungen auf. Die Petrischalen bleiben so lange offen stehen, bis das Lösungsmittel vollständig verdunstet ist. Anschliessend gibt man 25 Testtiere in die Petrischalen und bedeckt sie mit einem Glasdeckel.

Der Zustand der Tiere wird bis zu 6 Stunden fortlaufend und danach noch nach 24, 48 und 72 Stunden kontrolliert. Es wird diejenige Zeit ermittelt, die für eine 100%ige knock down-Wirkung erforderlich ist. Wird die $LT_{100}$ nach 72 Stunden nicht erreicht, wird der Prozentsatz der knock down gegangenen Testtiere festgestellt.

Konzentrationen der Wirkstoffe, Synergisten und Gemische und ihre Wirkung gehen aus der nachfolgenden Tabelle hervor.

| Wirkstoff Kennbuchstabe | + | Synergist-Nr. | Konzentrationen in % Wirkstoff | + | Synergist | $LT_{100}$ nach Minuten, bzw. % nach Minuten |
|---|---|---|---|---|---|---|
| B | + | 6 | 0,04 | + | 0,04 | 360′ = 50% |
| B | + | 1 | 0,008 | + | 0,008 | 120′ |
| B | + | 2 | 0,008 | + | 0,008 | 210′ |
| B | + | 3 | 0,04 | + | 0,04 | 105′ |
| B | + | 4 | 0,04 | + | 0,04 | 150′ |
| B | + | 5 | 0,008 | + | 0,008 | 120′ |
| C | + | 6 | 0,2 | + | 0,2 | 360′ = 80% |
| C | + | 1 | 0,04 | + | 0,04 | 105′ |
| C | + | 2 | 0,04 | + | 0,04 | 90′ |
| C | + | 3 | 0,2 | + | 0,2 | 120′ |
| C | + | 4 | 0,2 | + | 0,2 | 210′ |
| C | + | 5 | 0,04 | + | 0,04 | 90′ |
| D | + | 6 | 0,2 | + | 0,2 | 210′ |
| D | + | 2 | 0,2 | + | 0,2 | 90′ |
| D | + | 3 | 0,2 | + | 0,2 | 150′ |
| D | + | 4 | 0,2 | + | 0,2 | 180′ |
| D | + | 5 | 0,04 | + | 0,04 | 210′ |
| E | + | 6 | 0,2 | + | 0,2 | 45′ |
| E | + | 1 | 0,2 | + | 0,2 | 45′ |
| E | + | 4 | 0,2 | + | 0,2 | 45′ |
| E | + | 5 | 0,2 | + | 0,2 | 45′ |
| F | + | 6 | 0,04 | + | 0,04 | 120′ |
| F | + | 1 | 0,04 | + | 0,04 | 105′ |
| F | + | 2 | 0,04 | + | 0,04 | 75′ |
| F | + | 4 | 0,04 | + | 0,04 | 120′ |
| F | + | 5 | 0,04 | + | 0,04 | 90′ |
| G | + | 6 | 0,04 | + | 0,04 | 90′ |
| G | + | 1 | 0,04 | + | 0,04 | 75′ |
| G | + | 2 | 0,04 | + | 0,04 | 75′ |
| G | + | 4 | 0,04 | + | 0,04 | 90′ |
| G | + | 5 | 0,04 | + | 0,04 | 75′ |
| H | + | 6 | 0,0016 | + | 0,0016 | 60′ |
| H | + | 3 | 0,0016 | + | 0,0016 | 45′ |
| H | + | 4 | 0,0016 | + | 0,0016 | 45′ |
| I | + | 6 | 0,0016 | + | 0,0016 | 360′ = 95% |
| I | + | 1 | 0,0016 | + | 0,0016 | 75′ |
| I | + | 2 | 0,0016 | + | 0,0016 | 105′ |
| I | + | 3 | 0,0016 | + | 0,0016 | 105′ |
| I | + | 4 | 0,0016 | + | 0,0016 | 105′ |
| I | + | 5 | 0,0016 | + | 0,0016 | 90′ |

| Wirkstoff Kennbuchstabe | + | Synergist-Nr. | Konzentrationen in % Wirkstoff | + | Synergist | $LT_{100}$ nach Minuten, bzw. % nach Minuten |
|---|---|---|---|---|---|---|
| J | + | 6 | 1,0 | + | 1,0 | 360′ = 95% |
| J | + | 1 | 0,04 | + | 0,04 | 120′ |
| J | + | 3 | 0,2 | + | 0,2 | 240′ |
| J | + | 4 | 0,04 | + | 0,04 | 360′ |
| J | + | 5 | 0,04 | + | 0,04 | 210′ |
| K | + | 6 | 0,04 | + | 0,04 | 150′ |
| K | + | 1 | 0,04 | + | 0,04 | 90′ |
| K | + | 2 | 0,04 | + | 0,04 | 120′ |
| K | + | 4 | 0,04 | + | 0,04 | 105′ |
| K | + | 5 | 0,04 | + | 0,04 | 90′ |
| L | + | 6 | 0,008 | + | 0,008 | 105′ |
| L | + | 1 | 0,008 | + | 0,008 | 90′ |
| L | + | 3 | 0,008 | + | 0,008 | 90′ |
| L | + | 4 | 0,008 | + | 0,008 | 90′ |
| L | + | 5 | 0,008 | + | 0,008 | 90′ |
| M | + | 6 | 0,04 | + | 0,04 | 360′ = 90% |
| M | + | 1 | 0,04 | + | 0,04 | 180′ |
| M | + | 2 | 0,04 | + | 0,04 | 240′ |
| M | + | 3 | 0,04 | + | 0,04 | 150′ |

Die Herstellung der erfindungsgemäss verwendbaren Synergisten der Formel (I) erfolgt in bekannter Weise nach literaturbekannten Methoden und soll durch die folgenden Beispiele erläutert werden: (alle %-Angaben in der Beschreibung sind Gewichts-%, soweit nichts anderes angegeben ist):

Beispiel 1

3,4 g (0,11 Mol) Natriumhydrid 80%ig werden bei Raumtemperatur (20 ± 5°C) zu 75 ml Propargylalkohol gegeben; dann werden bei 25°C (16,7 g) (0,1 Mol) 2-Chlor-5-methylbenzothiazol zugetropft und das Reaktionsgemisch wird bei dieser Temperatur 15–20 Stunden gerührt. Überschüssiger Propargylalkohol wird unter vermindertem Druck abdestilliert; der Rückstand wird dann mit Wasser und Methylenchlorid geschüttelt, die organische Phase abgetrennt, mit Wasser gewaschen, getrocknet, filtriert und das Filtrat wird eingeengt. Das erhaltene 2-Propargyloxy-5-methylbenzthiazol hat einen Schmelzpunkt von 55°C.

Die Verbindungen der folgenden Beispiele können analog Beispiel 1 hergestellt werden.

| Beispiel Nr. | R′ | R″ | R″ | R‴ | X | Schmelzpunkt (°C) bzw. Siedepunkt (°C/Pa ‹Tour› |
|---|---|---|---|---|---|---|
| 2 | H | H | H | H | O | 132/1333 ‹10› |
| 3 | H | Cl | H | H | O | 72 |
| 4 | H | Cl | H | Cl | O | 91 |
| 5 | H | H | H | M | S | 53 |
| 6 | H | H | Cl | H | O | 148/400 ‹3› |

## Patentanspruch

Verwendung der Verbindungen der Formel (I)

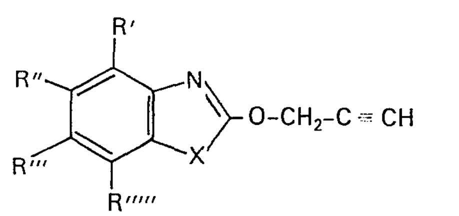

(I)

in welcher

X für Sauerstoff oder Schwefel steht,
als Synergisten in Schädlingsbekämpfungsmitteln, insbesondere zusammen mit anderen Insektiziden und Akariziden.

R′, R″, R′″und R″″ unabhängig voneinander für Wasserstoff, Halogen oder $C_1$-$C_4$-Alkyl stehen und

**Claim**

Use of the compounds of the formula (I)

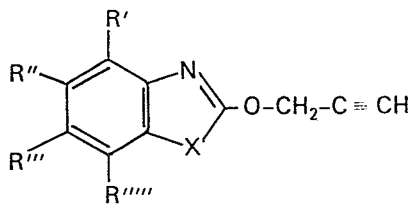

(I)

in which
R′, R″, R′″and R″″ independently of one another represent hydrogen, halogen or $C_1$-$C_4$-alkyl and

X represents oxygen or sulphur,
as synergists in agents for combating pests, in particular together with other insecticides and acaricides.

**Revendication**

Utilisation des composés de formule (I)

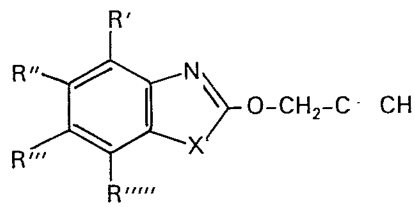

(I)

dans laquelle
R′, R″, R′″et R″″ représentent indépendamment les uns des autres l'hydrogène, un halogène ou un alkyle en $C_1$-$C_4$ et
X représente l'oxygène ou le soufre,
comme synergistes dans des agents antiparasitaires, en particulier en association avec d'autres insecticides et acaricides.